Europäisches Patentamt

European Patent Office

Office européen des brevets

(19)

(11) Numéro de publication : **0 509 906 A1**

# (12) DEMANDE DE BREVET EUROPEEN

(21) Numéro de dépôt : **92401045.7**

(51) Int. Cl.⁵ : **A61K 31/70**

(22) Date de dépôt : **15.04.92**

(30) Priorité : **16.04.91 GB 9108085**

(43) Date de publication de la demande :
**21.10.92 Bulletin 92/43**

(84) Etats contractants désignés :
**DE FR IT NL**

(71) Demandeur : **SOCIETE DE CONSEILS DE RECHERCHES ET D'APPLICATIONS SCIENTIFIQUES (S.C.R.A.S.)**
**51/53 rue du Docteur Blanche**
**F-75016 Paris (FR)**

(72) Inventeur : **Hovanessian, Ara G.**
**124 avenue Gal Leclerc**
**F-92340 Bourg la Reine (FR)**
Inventeur : **Deschamps de Paillette, Evelyne**
**60 avenue Théophile Gautier**
**F-75016 Paris (FR)**

(74) Mandataire : **Lachassagne, Jacques**
**Boite Postale 07**
**F-78430 Louveciennes (FR)**

(54) **Complexes de l'acide polyadenylique avec l'acide polyuridylique pour le traitement du SIDA.**

(57)     L'invention concerne une composition thérapeutique pour le traitement du Syndrome de l'Immuno Déficience Acquise (Sida) et les affections similaires, ladite composition comportant un principe actif associé à des diluants ou excipients pharmacologiquement acceptables, caractérisée en ce que la composition du principe actif comprend de 90 à 100 % du complexe Poly(A).Poly(U) optionellement associé à un autre agent anti-Sida agissant sur le virus VIH selon un mécanisme différent de celui du complexe Poly(A).Poly(U).

EP 0 509 906 A1

L'invention concerne des compositions pharmaceutiques contenant les complexes de l'acide polyadenylique avec l'acide polyuridylique, optionellement associés avec un agent anti-sida (Syndrome de l'Immuno Déficience Acquise ), destinées au traitement du Sida.

Le brevet français N° 2 622 586 décrit un procédé de préparation d'homopolymères et copolymères de polynucléotides et de leurs complexes. Bien que ces composés ne soient pas nouveaux, les procédés antérieurs n'étaient pas capables de fournir de tels complexes à un coût économiquement acceptable et sans impuretés toxiques ce qui les rendaient inappropriés pour un usage pharmacologique. Le procédé, décrit dans le brevet français ci-dessus, conduit à des produits d'une pureté suffisante pour un usage pharmacologique et nommément décrits pour le traitement des tumeurs.

Le complexe du Poly(A).Poly(U) a été décrit comme un agent anti-viral peu actif sur des souches de virus différentes de celles des rétrovirus responsables du Sida (cf "Effects of polynucleotides on monkeys and man", CLINICAL ASPECTS OF INTERFERONS, 1988, pages 319-331) ; par conséquent, il n'était pas évident que le Poly(A).Poly(U) puisse être un agent anti-rétroviral, les virus et rétrovirus étant considérés comme des espèces distinctes.

Selon l'invention, il a été prouvé que le complexe Poly(A).Poly(U) est aussi un agent inhibiteur puissant des différents Virus de l'Immuno-déficience Humaine (VIH) dans les cultures cellulaires, en bloquant l'entrée du virus. L'invention concerne donc une composition thérapeutique pour le traitement du Sida dans laquelle le principe actif contient du Poly(A).Poly(U). De préférence, le Poly(A).Poly(U) est préparé selon le procédé décrit dans le brevet français N° 2 622 586.

Il a été aussi prouvé que le complexe Poly(A).Poly(U) est efficace lorsqu'il est administré avec d'autres agents anti-Sida, tels que la 3'-azido-3'-déoxythymidine (AZT), la Didéoxyinosine (DDI) ou la Didéoxycytidine (DDC), car il accroît de façon synergique l'effet de ces derniers. En conséquence, l'invention concerne également une composition thérapeutique dont le principe actif contient du Poly(A).Poly(U) associé avec un autre agent anti-Sida.

En conséquence, cette invention concerne une composition thérapeutique pour le traitement du Syndrome de l'Immuno Déficience Acquise (Sida) et les affections similaires, ladite composition comportant un principe actif associé à des diluants ou excipients pharmacologiquement acceptables, caractérisée en ce que la composition du principe actif comprend de 90 à 100 % du complexe Poly(A).Poly(U) optionellement associé à un autre agent anti-Sida agissant sur le virus VIH selon un mécanisme différent de celui du complexe Poly(A).Poly(U).

De préférence, ladite composition du principe actif comprend de 99,00 à 99,99 % du complexe Poly(A).Poly(U) et de 1,00 à 0,01 % de l'autre agent anti-Sida.

Selon un mode de réalisation de l'invention, l'autre agent anti-Sida agissant sur le virus VIH, est choisi parmi l'AZT, la DDI ou la DDC.

L'intérêt du Poly(A).Poly(U) dans le traitement du Sida, ressort clairement des différentes expériences décrites ci-dessous.

## CYTOPATHOLOGIE

Deux lots de cellules CEM (cellules T riches en récepteurs CD4), infectées par le virus de l'Immuno Déficience Humaine VIH-1 Bru (Lai) sont traitées avec du Poly(A).Poly(U), 24 heures après l'infecrion. Deux lots similaires de cellules CEM infectées par le VIH, sont utilisées comme contrôle et non traitées. Les cultures cellulaires sont régulièrement inspectées sous microscope, afin d'observer l'évolution des effets cytopathiques (fusion de cellules et formation de Syncitia), résultant de l'action du VIH.

Le protocole expérimental est le suivant: $5 \times 10^6$ cellules CEM sont incubées avec 1 ml de surnageant contenant du VIH, avec une reverse transcriptase d'une activité de $1,0 \times 10^6$ cpm. Une heure plus tard, les cellules sont centrifugées et le résidu cellulaire ($0,5 \times 10^6$ cellules) est mis en suspension dans un milieu RMP1 contenant 10 % de sérum foetal et 2 µg/ml de polybrène. 24 heures plus tard, le Poly(A).Poly(U) est ajouté à une concentration de 200 µg/ml. Les cultures cellulaires sont ainsi maintenues sans ajout de Poly(A).Poly(U).

Les résultats sont les suivants :

|  | Jour 6 | Jour 7 | Jour 8 | Jour 11 |
|---|---|---|---|---|
| Contrôles | ++ | ++++ | ++++ | cellules mortes |
| Cellules traitées | - | + | + | ++ |

Le symbole "+" indique l'apparition des effets cytopathiques ; le nombre de ces symboles utilisés est une quantification approximative. Ces résultats montrent que les effets cytopathiques sont réduits de façon significative par le traitement.

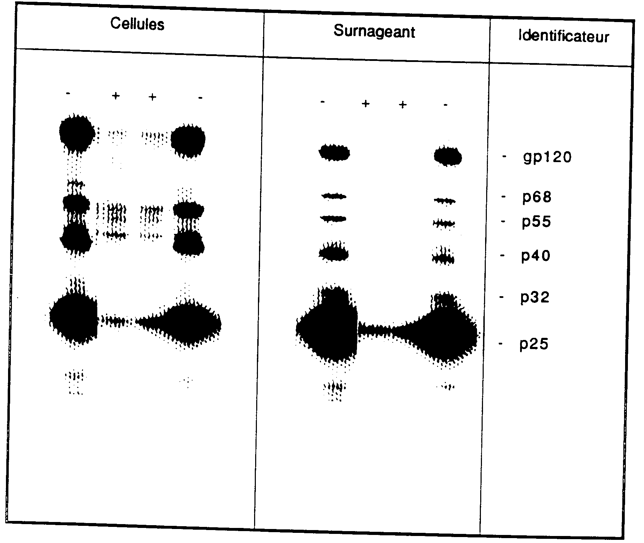

Les cultures sont préparées comme décrit ci-dessus et, pendant la nuit du jour 7 à 8, elles sont incubées avec de la $^{35}$S-méthionine, pour une analyse électrophorétique du SDS des protéines virales synthétisées à la fois dans la masse cellulaire et dans le surnageant. Ces résultats sont présentés sur le dessin annexé. Sur ce dessin, "-" indique un échantillon de contrôle non traité et "+" un échantillon de culture traitée avec du Poly(A).Poly(U). Les identificateurs sur la droite des taches sont les suivants :

gp 120     la protéine de l'enveloppe
p 68       la reverse transcriptase
p 55       le précurseur de la protéine du noyau
p40        le précurseur de la protéine du noyau, partiellement clivée
p 32       l'endonucléase
p25        la protéine principale du noyau

Les résultats montrent une réduction presque totale de la présence de protéines virales dans les cellules et les surnageants de cultures traitées avec le Poly(A).Poly(U).

La quantité d'antigènes p24/p25 dans le surnageant, est mesurée 8 jours après l'infecrion, au moyen du test ELISA.

| Contrôles | Cellules traitées |
|---|---|
| 1,01 µg/ml | 0,03 µg/ml |
| 1,27 µg/ml | 0,07 µg/ml |

Ceci montre une suppression de 96,5 % de la production de VIH dans les cultures traitées avec le Poly(A).Poly(U).

L'action du Poly(A).Poly(U) sur l'inhibition de la production de VIH dans des cellules en culture, a été confirmée par les expériences ci-après.

1. Effets de la variation de la dose de Poly(A).Poly(U) :

Les cellules CEM sont infectées par le VIH. 24 heures plus tard, différentes concentrations de Poly(A).Poly(U) sont ajoutées. La production de virus dans le surnageant de cultures, est déterminée au jour 5 après l'infecrion, en quantifiant la quantité d'antigènes p24/p25 présents. Les cellules utilisées comme contrôles et qui ont été infectées mais non traitées par le Poly(A).Poly(U), éclatent.

Les résultats obtenus sont les suivants :

| Poly(A).Poly(U) (µg/ml) | p24/p25 (µg/ml) |
|---|---|
| 0 | 1,10 |
| 25 | 0,53 |
| 50 | 0,14 |
| 100 | 0,10 |
| 200 | 0,07 |

2. Effets de traitement répété :

Le mode opératoire est identique à celui décrit dans la première expérience, mais l'addition de Poly(A).Poly(U) se fait à une concentration de 200 µg/ml, 24 heures et 96 heures après l'infection. Un contrôle, non traité, est également conservé ; comme précédemment, les cellules du contrôle se détruisent au jour 5.

La concentration (µg/ml) de p24/p25, aux jours 4, 5, 6 et 7 après infection, est mesurée sur le contrôle non traité et sur les cellules traitées aux jours 1 et 4.

Les résultats obtenus sont les suivants :

| | p24/p25 (µg/ml) | | | |
| --- | --- | --- | --- | --- |
| | Jour 4 | Jour 5 | Jour 6 | Jour 7 |
| Contrôles | 0,72 | 1,25 | | |
| Cellules traitées | 0,05 | 0,10 | 0,20 | 0,26 |

3. L'effet du Poly(A).Poly(U) se fait lors de la première étape de l'infection virale :

Les cellules, infectées par le VIH, sont traitées avec 200 µg/ml de Poly(A).Poly(U) à différents moments, avant, pendant, et après l'infection du VIH. Au jour 6 après l'infection, le nombre de Syncitia est compté. Au jour 7, le niveau de p25 dans le milieu de culture, est mesuré.

| Poly(A).Poly(U) | Syncitia (%) | p25 (ng/ml) |
| --- | --- | --- |
| Témoin (non traité) | 90 | 1320 |
| Cellules traitées : | | |
| . 3 jours avant | 85 | 1215 |
| . 2 jours avant | 72 | 965 |
| . 1 jour avant | 45 | 280 |
| . 1 heure avant | <5 | 6 |
| . avec le VIH | <5 | 5 |
| . 1 heure après | 10 | 110 |
| . 4 heures après | 10 | 120 |

ETUDE DE L'EMPLOI DU POLY(A).POLY(U) AVEC L'AZT :

Les cellules sont infectées par le VIH, 24 heures avant un unique traitement :
(a) de Poly(A).Poly(U) seul, à une concentration de 100 µg/ml;
(b) d'AZT seul, aux différentes concentrations de 100, 250, 1000, 5000 et 10 000 ng/ml (5 échantillons);
(c) d'AZT aux différentes concentrations de 100, 250, 1000, 5000 et 10 000 ng/ml, chacune avec une concentration de Poly(A).Poly(U) de 100 µg/ml (5 échantillons).

De plus, un témoin, non traité, sert de référence ; la culture cellulaire du contrôle meurt progressivement après le jour 7 ce qui explique qu'aucune valeur n'apparaisse dans les colonnes des jours 12 et 14.

Le niveau de l'antigène p25 dans les surnageants, est mesuré aux jours 7, 12 et 14 après infection. Les résultats, exprimés en µg/ml, sont les suivants :

| | p25 | | |
| | Jour 7 | Jour 12 | Jour 14 |
|---|---|---|---|
| Témoin | 1250 | | |
| Poly(A).Poly(U) | 135 | 980 | 1200 |
| AZT 100 | 38 | 380 | 1200 |
| AZT 100 Poly(A).Poly(U) | 16 | 75 | 900 |
| AZT 250 | 18 | 170 | 1200 |
| AZT 250 Poly(A).Poly(U) 100 | 5 | 40 | 840 |
| AZT 1000 | <5 | 52 | 590 |
| AZT 1000 Poly(A).Poly(U) 100 | <5 | 17 | 200 |
| AZT 5000 | <5 | 27 | 275 |
| AZT 5000 Poly(A).Poly(U) 100 | <5 | 10 | 48 |
| AZT 10 000 | <5 | 20 | 220 |
| AZT 10 000 Poly(A).Poly(U) 100 | <5 | 8 | 39 |

Les résultats montrent que l'association AZT/Poly(A).Poly(U) est plus efficace que l'un d'eux pris séparément. En effet, le Poly(A).Poly(U) présente un effet inhibiteur sur le VIH. Ceci a été montré par les tests ci-dessous:

A: la synthèse métabolique des protéines virales dans les cellules infectées

B : l'activité de la reverse transcriptase dans le milieu de culture des cellules infectées

C: la quantité de la protéine principale du noyau p25 du VIH dans le milieu de culture des cellules infectées

D : la fusion de cellules

L'action inhibitrice du Poly(A).Poly(U) sur différents et isolats du VIH :

Dans toutes les expériences présentées ci-dessus, on a urilisé l'isolat VIH-1 Bru Lai. Afin de prouver que l'action inhibitrice du Poly(A).Poly(U) ne se limite pas à la seule espèce VIH-1 utilisée ci-dessus, d'autres espèces de VIH-1 telles que ELI (31) et deux espèces différentes du VIH-2, ROD et EHO, ont été testées. Le Poly(A).Poly(U), ajouté 6 heures avant l'infestation de cellules CEM avec ces virus, conduit à plus de 90 % d'inhibition de la production virale, ce qui prouve que l'action démontrée s'applique à toutes les espèces de VIH actuellement connues.

Ces expérimentations démontrent une puissante action anti-rétrovirale. Il apparaît que le Poly(A).Poly(U) semble agir au niveau de la pénétration du virus dans la cellule, alors que l'AZT agit au niveau de la transcription intra-cellulaire. Utiliser le traitement combinant le Poly(A).Poly(U) avec un autre agent anti-retroviral donne de bien meilleurs résultats que chacun d'eux seul et même que l'addition des activités de chacun d'eux aux mêmes doses.

Présentation - Posologie:

Dans tous ces tests, le Poly(A).Poly(U), à la dose de 200 µg/ml, exerce une action inhibitrice qui peut être estimée entre 85 et 90 %. Une quantité de 100 à 4000 mg pour une injection unique chez l'homme est souhaitable ; des doses, injectées chez l'homme, peuvent être efficaces entre 150 et 1000 mg. L'effet maximal est obtenu par un traitement administré le plus proche possible de l'infestation, mais un traitement avant ou après l'infestation est également efficace. Le traitement peut être répété à des intervalles de 3 à 5 jours. L'administration se fait par injection I.V. des principes actifs dissous en milieu aqueux.

Pour la préparation de la solution à injecter, il est prévu, de préférence, de dissoudre de 0,2 à 1,0 g de principe actif dans 100 ml de milieu aqueux. Les autres agents tels que l'AZT, la DDI et la DDC, sont, le cas échéant, ajoutés dans les proportions indiquées plus haut, à la composition ainsi préparée. La composition d'une solution aqueuse prête à l'emploi, pourrait être conforme à ce qui est décrit ci-après.

| | |
|---|---|
| $NaH_2PO_4-H_2O$ | 0,100 g |
| NaCl | 0,068 g |
| NaOH | pour neutraliser (pH 7) |
| $H_2O$ | q.s.p. pour 100 ml |

Dans 100 ml de solution aqueuse, les constituants suivants sont dissous:

| | |
|---|---|
| Poly(A).Poly(U) | 0,40 g |
| Mannitol | 1,84 g |
| NaCl | 0,48 g |

Si on souhaite ajouter un ou plusieurs autres agents anti-sida, ces derniers sont ajoutés de façon cumulative dans la solution aqueuse, c'est à dire que rien n'est retiré pour tenir compte de l'addition du second agent. La quantité désirée de cet agent, par exemple l'AZT, est de 0,04 mg à 4 mg pour 100 ml de solution aqueuse. Pour administrer 150 mg de Poly(A).Poly(U) associé à 1,5 mg d'AZT, il suffira d'injecter 37,5 ml de solution aqueuse telle que décrite ci-dessus.

## Revendications

1. Composition thérapeutique pour le traitement du Syndrome de l'Immuno Déficience Acquise (Sida) et les affections similaires, ladite composition comportant un principe actif associé à des diluants ou excipients pharmacologiquement acceptables, caractérisée en ce que la composition du principe actif comprend de 90 à 100 % du complexe Poly(A).Poly(U) optionnellement associé à un autre agent anti-Sida agissant sur le virus VIH selon un mécanisme différent de celui du complexe Poly(A).Poly(U).

2. Composition thérapeutique selon la revendication 1, ladite composition caractérisée en ce que la composition du principe actif comprend de 90 à 99,99 % du complexe Poly(A).Poly(U) et de 10 à 0,01% de l'autre agent anti-Sida.

3. Composition thérapeutique selon les revendications 1 et 2, ladite composition caractérisée en ce que la composition du principe actif comprend de 99,00 à 99,99 % du complexe Poly(A).Poly(U) et de 1,00 à 0,01% de l'autre agent anti-Sida.

4. Composition thérapeutique selon les revendications 1 à 3, dans laquelle l'agent anti-Sida agissant sur le virus VIH selon un mécanisme différent de celui du complexe Poly(A).Poly(U), est choisi parmi la 3'-azido-3'-déoxythymidine (AZT), la Didéoxyinosine (DDI) ou la Didéoxycytidine (DDC).

| Cellules | Surnageant | Identificateur |
|----------|------------|----------------|
|          |            | -  gp120 |
|          |            | -  p68 |
|          |            | -  p55 |
|          |            | -  p40 |
|          |            | -  p32 |
|          |            | -  p25 |

Office européen
des brevets

**RAPPORT DE RECHERCHE EUROPEENNE**

Numero de la demande

EP 92 40 1045

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,X | ABSTRACTS OF THE VII INTERNATIONAL CONFERENCE ON AIDS: SCIENCE CHALLENGING AIDS, Florence, 16-21 juin 1991, Edited by Istituto Superiore di Sanita, Rome, GR, page 113, abrégé no. W.A.1084; A. HOVANESSIAN et al.: "Antiviral activity of poly(A).poly(U) against HIV in vitro"<br>* Abrégé *<br>--- | 1-4 | A 61 K 31/70 |
| P,X | AIDS RESEARCH AND HUMAN RETROVIRUSES, vol. 8, no. 2, février 1992, pages 285-290; A.G. LAURENT-CRAWFORD et al.: "Antiviral action of polyadenylic-polyuridylic acid against HIV in cell cultures"<br>* En entier *<br>--- | 1-4 | |
| Y | EP-A-0 286 224 (HEM RESEARCH, INC.)<br>* Revendications *<br>--- | 1-4 | |
| Y | EP-A-0 300 680 (HEM RESEARCH, INC.)<br>* En entier, spécialement revendications 9,13 *<br>--- | 1-4 | **DOMAINES TECHNIQUES RECHERCHES (Int. Cl.5)**<br><br>A 61 K |
| Y | EP-A-0 350 151 (HEM RESEARCH, INC.)<br>* En entier, spécialement revendications 5,6 *<br>--- | 1-4 | |
| Y | CELL BIOLOGY INTERNATIONAL REPORTS, vol. 14, no. 12, décembre 1990, pages 1075-1084; J.M. JAMISON et al.: "Potentiation of the antiviral activity of poly r(A-U) by xanthene dyes"<br>* En entier *<br>---         -/- | 1-4 | |

Le présent rapport a été établi pour toutes les revendications

| Lieu de la recherche | Date d'achèvement de la recherche | Examinateur |
|---|---|---|
| LA HAYE | 03-07-1992 | ORVIZ DIAZ P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

Office européen
des brevets

**RAPPORT DE RECHERCHE EUROPEENNE**

Numero de la demande

EP  92 40 1045

## DOCUMENTS CONSIDERES COMME PERTINENTS

| | Catégorie | Citation du document avec indication, en cas de besoin, des parties pertinentes | Revendication concernée | CLASSEMENT DE LA DEMANDE (Int. Cl.5) |
|---|---|---|---|---|
| 2 | Y | THE JOURNAL OF IMMUNOLOGY, vol. 117, no. 2, août 1976, pages 423-427, The Williams & Wilkins Co., US; I.H. HAN et al.: "Regulation of the immune system by synthetic polynucleotides. VI. Amplification of the immune response in young and aging mice"<br>* En entier *<br>--- | 1-4 | |
| 2 | Y | LA NOUVELLE PRESSE MEDICALE, vol. 9, no. 23, 24 mai 1980, pages 1633-1636; G. RENOUX: "L'immunopharmacologie au service de l'immunothérapie"<br>* En entier *<br>--- | 1-4 | |
| 1 | D,Y | M. REVEL: "Clinical Aspects of Interferons", 1988, pages 319-331, Kluwer Academic Publishers, Boston, US, chapitre 24; H.B. LEVY: "Effects of polynucleotides on monkeys and man"<br>* Pages 319-325,330 *<br>----- | 1-4 | DOMAINES TECHNIQUES RECHERCHES (Int. Cl.5) |

Le présent rapport a été établi pour toutes les revendications

| Lieu de la recherche | Date d'achèvement de la recherche | Examinateur |
|---|---|---|
| LA HAYE | 03-07-1992 | ORVIZ DIAZ P. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons

& : membre de la même famille, document correspondant

EPO FORM 1503 03.82 (P0402)